# EUROPEAN PATENT SPECIFICATION

(11) **EP 2 412 810 B1**
(45) Date of publication and mention of the grant of the patent: **08.07.2015**
(21) Application number: 10756136.7
(22) Date of filing: 24.03.2010
(51) Int. Cl.: C12N 15/85

(54) **METHOD FOR AMPLIFICATION AND HIGH-LEVEL EXPRESSION OF TARGET GENE IN MAMMALIAN CELL, AND KIT FOR ACHIEVING THE METHOD**
VERFAHREN ZUR AMPLIFIKATION UND HOCHGRADIGEN EXPRESSION EINES ZIELGENS IN SÄUGERZELLEN UND KIT ZUR DURCHFÜHRUNG DIESES VERFAHRENS
PROCÉDÉ D'AMPLIFICATION ET D'EXPRESSION DE HAUT NIVEAU D'UN GÈNE CIBLE DANS UNE CELLULE MAMMALIENNE, ET KIT POUR RÉALISER LE PROCÉDÉ

(30) Priority: 27.03.2009 JP 2009080153
(43) Date of publication of application: 01.02.2012
(73) Proprietor: Hiroshima University, Hiroshima 7398511 (JP)
(72) Inventor: SHIMIZU, Noriaki, Hiroshima 739-0046 (JP)
(74) Representative: Cabinet Plasseraud
(86) International application number: PCT/JP2010/055123
(87) International publication number: WO 2010/110340

(56) References cited:
- JP-A- 2000 201 680
- JP-A- 2006 055 175
- JP-A- 2007 312 655
- JP-T- 2004 506 428
- SHIMIZU NORIAKI ET AL: "Amplification of plasmids containing a mammalian replication initiation region is mediated by controllable conflict between replication and transcription", CANCER RESEARCH, vol. 63, no. 17, 1 September 2003 (2003-09-01), pages 5281-5290, XP002458348, ISSN: 0008-5472
- TOSHIHIKO HASHIZUME ET AL: "Dissection of mammalian replicators by a novel plasmid stability assay", JOURNAL OF CELLULAR BIOCHEMISTRY, vol. 101, no. 3, 1 January 2007 (2007-01-01), pages 552-565, XP055060119, ISSN: 0730-2312, DOI: 10.1002/jcb.21210
- MCWINNEY, C. ET AL.: 'AUTONOMOUS REPLICATIONS OF A DNA FRAGMENT CONTAINING THE CHROMOSOMAL REPLICATION ORIGIN OF THE HUMAN C-MYC GENE' NUCLEIC ACIDS RESEARCH vol. 18, no. 5, 1990, pages 1233 - 1242, XP001526451
- MARGOLSKEE, R.F. ET AL.: 'EPSTEIN-BARR VIRUS SHUTTLE VECTOR FOR STABLE EPISOMAL REPLICATION OF COMPLEMENTARY DNA EXPRESSION LIBRARIES IN HUMAN CELLS' MOLECULAR AND CELLULAR BIOLOGY vol. 8, no. 7, 1988, pages 2837 - 2847, XP002367031
- BAIKER, A. ET AL.: 'Mitotic stability of an episomal vector containing a human scaffold/ matrix-attached region is provided by association with nuclear matrix' NATURE CELL BIOLOGY vol. 2, no. 3, 2000, pages 182 - 184, XP008018671
- BERTONI, L. ET AL.: 'Telomeric and nontelomeric (TTAGGG)-n sequences in gene amplification and chromosome stability' GENOMICS vol. 24, no. 1, 1994, pages 53 - 62, XP024796720
- LUNDBERG, G. ET AL.: 'Binomial Mitotic Segregation of MYCN-Carrying Double Minutes in Neuroblastoma Illustrates the Role of Randomness in Oncogene Amplification' PLOS ONE vol. 3, no. 8, 2008, XP008153193

## Description

### Technical Field

The present invention relates to a method for highly amplifying a target gene in a mammalian cell to perform high-level expression, and a kit used for performing such a method. More specifically, the present invention relates to means which allows for amplifying a target gene without the target gene being incorporated into a chromosome of a host cell, in amplifying a preferable gene by a "high gene amplification system" developed by the inventor of the present invention. According to the present invention, it is possible to highly amplify a target gene in a mammalian cell, outside a chromosome of a host cell.

### Background Art

The inventor of the present invention found that, simply by transferring a plasmid (called "IR/MAR vector" or "IR/MAR plasmid") including a mammalian replication initiation region (IR; Initiation Region) and a mammalian nuclear matrix attachment region (MAR; Matrix Attachment Region) to a human-derived cancer cell (COLO 320 colon cancer cell strain and HeLa cell strain) by use of a lipofection method and then selecting the cell by utilizing a drug resistance gene (Blasticidin or Neomycin) being present on the plasmid, it is possible to:
(1) increase a copy number of a gene inside the cell up to approximately 10,000 copies, which gene encodes a protein to be expressed (hereinafter, the gene is referred to as "target gene", as necessary), and
(2) highly amplify the target gene in either cases where the target gene and the IR/MAR vector are transferred in a single gene construct (cis) or where the target gene and the IR/MAR vector are transferred respectively in different gene constructs (trans) (e.g., see Patent Literature 1 and Non Patent Literature 1).

Based on the findings, the inventor of the present invention accomplished a system (hereinafter called "high gene amplification system"), which makes it possible to amplify the target gene up to approximately 10,000 copies, simply by performing the steps of: transferring the IR/MAR vector and the target gene to the mammalian cell (e.g., human-derived cancer cell (COLO 320 colon cancer cell strain and HeLa cell strain) and Chinese hamster ovary cell (CHO cell)) by use of the lipofection method; and selecting the cell by utilizing the drug resistance gene (Blasticidin or Neomycin) being present on the plasmid. The inventor of the present invention further progressed with their studies regarding the high gene amplification system, and has disclosed various findings thereof (e.g. see Patent Literatures 2 to 8).

The gene amplified by the high gene amplification system is present inside the host cell nucleus in the form of a DM (double minute chromosome), which is an enormous extrachromosomal circular DNA, or in the form of a HSR (homogeneously staining region), which is an enormous structure incorporated into the chromosome of the host cell. In the mammalian cell in most cases, the target gene amplifies as the HSR. However, in addition that the HSR is a structure incorporated into the chromosome of the host cell, the target gene is of a highly repeated structure. Hence, the target gene is effected by translation inhibition in a repetitive-sequence-dependent manner. Accordingly, this caused a problem that an expressed amount of protein from the target gene does not increase in proportion to a gene amplified amount.

The form of an amplified structure is the HSR also in a method currently widely used as a bulk production system of protein, which method amplifies a DHFR gene and a target gene together in a CHO cell. Therefore, this method also has the problem that the number of amplified genes copies is not proportional to the amount of expressed protein.

Meanwhile, it was made clear by studies conducted by the inventor of the present invention that the amount of protein expressed from a target gene increases in proportion to the number of amplified gene copies if the target gene is amplified as the DM, i.e. outside the chromosome of the host cell, or alternatively, is amplified by being incorporated into the DM (see Patent Literature 2).

Accordingly, the inventor of the present invention independently found a method of controlling an amplification form of the gene, as the DM or HSR, and has made the method available to the public (see Patent Literature 6). The method disclosed in Patent Literature 6, in amplifying a gene by the high gene amplification system, (a) carries out a transferring step and a selecting step in a state in which transcription activity of a transcription activity-adjusting promoter is activated in a case where the target gene is to be amplified as a small-sized amplification region on a double minute chromosome or in a homogeneously staining region, or (b) carries out the transferring step and the selecting step in a state in which the transcription activity of the transcription activity-adjusting promoter is inactivated in a case where the target gene is to be amplified as a large-sized amplification region in the homogeneously staining region of the chromosome.

Moreover, the inventor of the present invention found that the target gene can be highly amplified in the form of the DM, that is, amplified outside the chromosome, by having the form of the IR/MAR vector as a straight-chain and as having at least one of ends of the IR/MAR vector be of a hairpin structure. The inventor has filed an international patent application of this finding (see Patent Literature 9).

### Citation List

### Patent Literature

Patent Literature 1
   Japanese Patent Application Publication, Tokukai, No. 2003-245083 A (Publication Date: September 2, 2003)
Patent Literature 2
   International Publication No. 2006/054561 brochure (International Publication Date: May 26, 2006)
Patent Literature 3
   Japanese Patent Application Publication, Tokukai, No. 2004-337066 A (Publication Date: May 15, 2004)
Patent Literature 4
   Japanese Patent Application Publication, Tokukai, No. 2006-55175 A (Publication Date: March 2, 2006)
Patent Literature 5
   Japanese Patent Application Publication, Tokukai, No. 2006-320332 A (Publication Date: November 30, 2006)
Patent Literature 6
   Japanese Patent Application Publication, Tokukai, No. 2007-312655 A (Publication Date: December 6, 2007)
Patent Literature 7
   Japanese Patent Application Publication, Tokukai, No. 2007-312656 A (Publication Date: December 6, 2007)
Patent Literature 8
   International Publication No. 2008/023671 brochure (International Publication Date: February 28, 2008)
Patent Literature 9
   International Publication No. 2009/048024 brochure (International Publication Date: April 16, 2009)

### Non Patent Literature

Non Patent Literature 1
Noriaki Shimizu, et al. (2001) Plasmids with a Mammalian Replication Origin and a Matrix Attachment Region Initiate the Event Similar to Gene Amplification. Cancer Research vol.61, no. 19, p6987-6990.

### Summary of Invention

### Technical Problem

An object of the present invention, in order to further improve the high gene amplification system, is to provide novel means for amplifying a target gene in a form of the DM, namely, outside a chromosome of a host cell (mammalian cell), with a high probability, in a case where the target gene is amplified with use of an IR/MAR vector. If it is possible to amplify the target gene within the host cell (mammalian cell) in the form of the DM, less effect is given on the target gene by the foregoing expression inhibition, thereby allowing for obtaining an expressed amount of protein in proportion to the number of amplified copies of the target gene. Consequently, it can be said that a large amount of protein (referred to as "target protein"), which is encoded by the target gene, is finally achieved.

### Solution to Problem

The inventor of the present invention carried out diligent studies to solve the foregoing problem, and as a result found that a target gene can be amplified in a form of the DM, that is, outside the chromosome of the host cell, by concurrently transferring the IR/MAR vector, the target gene, and the polynucleotide including a telomere repetitive sequence, to a host cell (mammalian cell). This accomplished the present invention. Namely, the present invention includes the following inventions in order to attain the above objects.

A method according to the present invention is a method of amplifying a target gene outside a chromosome of a mammalian cell, the method including the step of: transferring, concurrently to a mammalian cell, (i) a vector including (a) a mammalian replication initiation region functioning in a mammalian cell and (b) a nuclear matrix attachment region functioning in a mammalian cell, (ii) a target gene, and (iii) a polynucleotide including a telomere repetitive sequence.

In the method according to the present invention, the telomere repetitive sequence may be a base sequence consisting of a base sequence of TTAGGG or TTAGGC being repeated several times.

The method of the present invention may be a method wherein the mammalian replication initiation region derives from a replication initiation region of a locus selected from the group consisting of a c-myc locus, a dihydrofolate reductase locus, and a ß-globin locus.

The method according to the present invention may be a method wherein the nuclear matrix attachment region derives from a nuclear matrix attachment region of a region selected from the group consisting of an Igκ locus, an SV40 early region, and a dihydrofolate reductase locus.

The method of the present invention may be a method wherein the target gene and the vector are transferred to the mammalian cell in such a manner that the target gene and the vector are arranged in cis.

The method of the present invention may be a method wherein the target gene and the vector are transferred to the mammalian cell in such a manner that the target gene and the vector are arranged in trans.

The method of the present invention may further include the step of: selecting a transformed cell after the step of transferring.

On the other hand, a transformed cell according to the present invention is a mammalian cell to which (i) a vector including (a) a mammalian replication initiation region functioning in a mammalian cell and (b) a nuclear matrix attachment region functioning in a mammalian cell, (ii) a target gene, and (iii) a polynucleotide including a telomere repetitive sequence are concurrently transferred, the target gene being amplified outside a chromosome of the mammalian cell.

The transformed cell according to the present invention may be configured in such a manner that the telomere repetitive sequence is a base sequence consisting of a base sequence of TTAGGG or TTAGGC being repeated several times.

The transformed cell according to the present invention may be configured in such a manner that the mammalian replication initiation region derives from a replication initiation region of a locus selected from the group consisting of a c-myc locus, a dihydrofolate reductase locus, and a β-globin locus.

The transformed cell according to the present invention may be configured in such a manner that the nuclear matrix attachment region derives from a nuclear matrix attachment region of a region selected from the group consisting of an Igκ locus, an SV40 early region, and a dihydrofolate reductase locus.

Meanwhile, a method according to the present invention of expressing a target gene, includes the step of: culturing a transformed cell according to the present invention.

Moreover, a kit according to the present invention is a kit for amplifying a target gene outside a chromosome of a mammalian cell, the kit comprising: a vector including (a) a mammalian replication initiation region functioning in a mammalian cell and (b) a nuclear matrix attachment region functioning in a mammalian cell; and a polynucleotide including a telomere repetitive sequence.

The kit according to the present invention is configured in such a manner that the telomere repetitive sequence may be a base sequence consisting of a base sequence of TTAGGG or TTAGGC being repeated several times.

The kit according to the present invention may be configured in such a manner that the mammalian replication initiation region derives from a replication initiation region of a locus selected from the group consisting of a c-myc locus, a dihydrofolate reductase locus, and a β-globin locus.

The kit according to the present invention may be configured in such a manner that the nuclear matrix attachment region derives from a nuclear matrix attachment region of a region selected from the group consisting of an Igκ locus, an SV40 early region, and a dihydrofolate reductase locus.

### Advantageous Effects of Invention

As described above, with the present invention, it is possible to amplify a target gene in the form of a DM, namely outside the chromosome of the mammalian cell, in amplifying the target gene by the high gene amplification system with use of the IR/MAR vector. Furthermore, according to the present invention, it is possible to obtain a transformed cell group in which the target gene is amplified in the form of the DM by a high probability. In the Examples later described, approximately 80 % of the obtained transformed cells had the target gene amplified in the form of the DM.

If it is possible to obtain a transformed cell that can amplify a target gene in the form of the DM by a high probability, it is possible to obtain an expressed amount of protein from the target gene in proportion to the number of amplified copies of the target gene. Hence, the present invention brings about an effect that a protein ("target protein") encoded by a target gene can be produced in bulk amounts.

### Brief Description of Drawings

Fig. 1

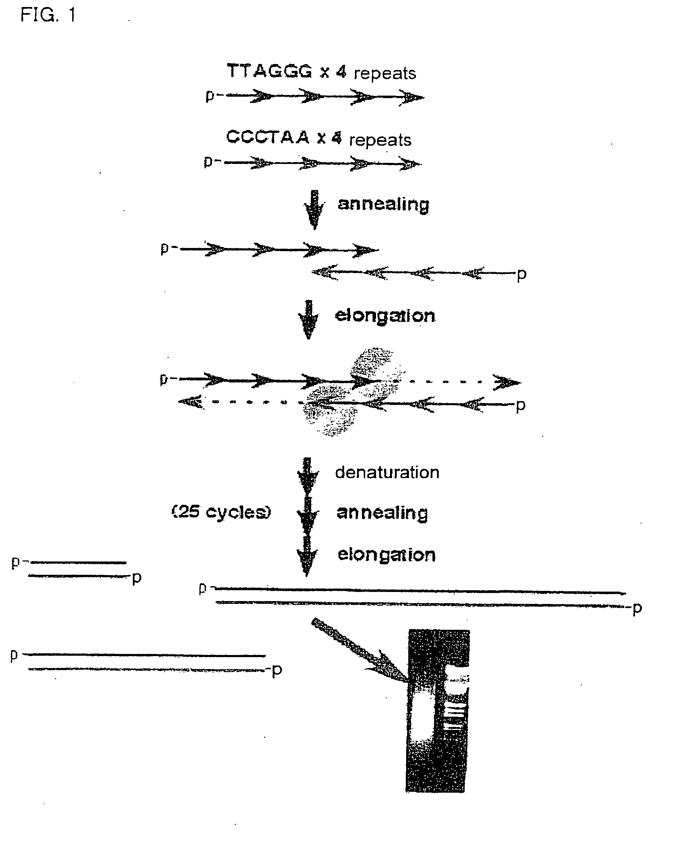

   Fig. 1 is a view schematically illustrating how a double stranded DNA used in Examples and Comparable Examples is prepared, which double stranded DNA is made of a vertebrate-derived telomere repetitive sequence.
Fig. 2

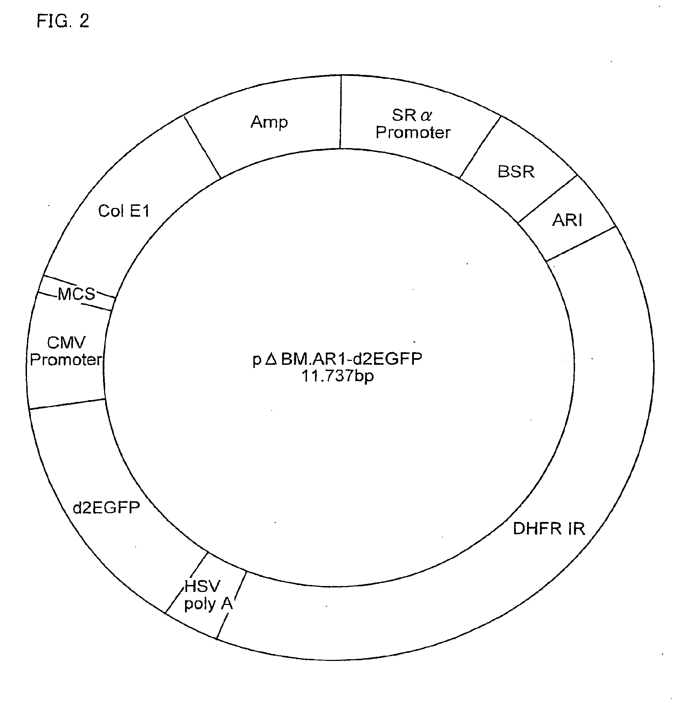

   Fig. 2 is a view illustrating a structure of pΔBM.AR1-d2EGFP, which is an IR/MAR vector used in Examples and Comparative Examples.
Fig. 3

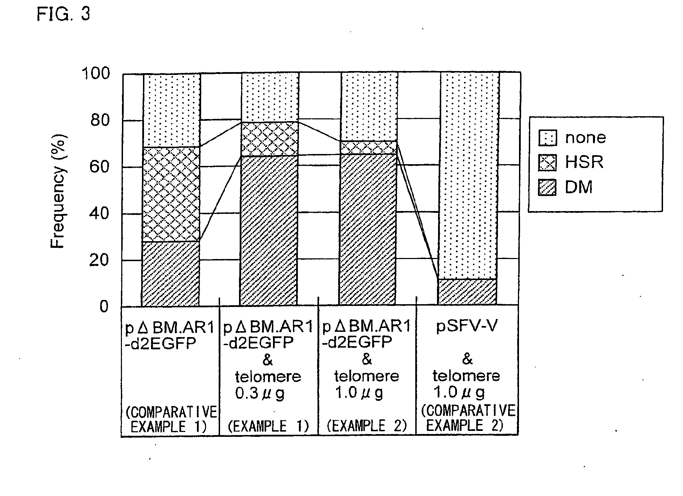

   Fig. 3 is a view showing a result of comparing a frequency of various gene amplification forms (HSR, DM) included in various polyclonal populations, by preparing a metaphase chromosome sample from each of polyclonal populations obtained in Example 1, Example 2, Comparative Example 1, and Comparative Example 2, and carrying out FISH with use of a probe prepared from a transferred plasmid DNA.
Fig. 4

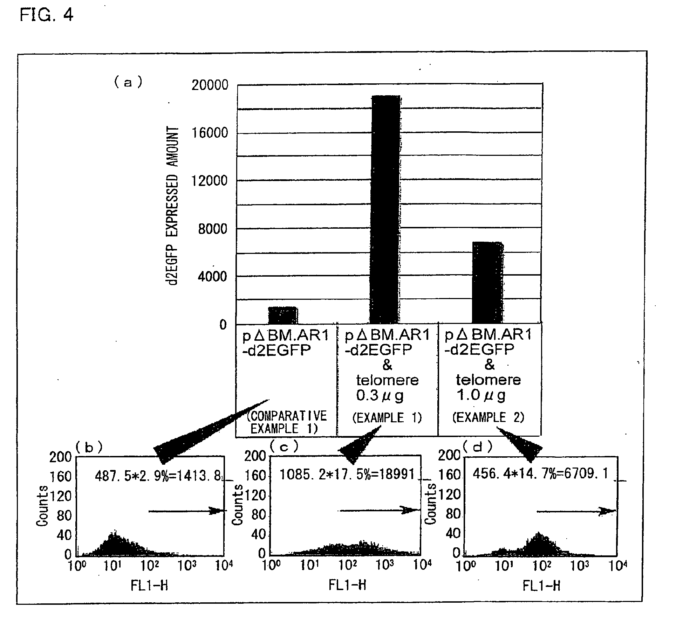

   Fig. 4 is a view showing a result of detecting, from polyclonal populations obtained in Comparative Example 1, Example 1, and Example 2, expression of d2EGFP by flow cytometry; (a) thereof shows a result of measuring an expressed amount of d2EGFP for Comparative Example 1, and Examples 1 and 2, (b) thereof shows a result of measuring an intensity of fluorescence of d2EGFP for Comparative Example 1 by flow cytometry, (c) thereof shows a result of measuring an intensity of fluorescence d2EGFP for Example 1 by flow cytometry, and (d) thereof shows a result of measuring an intensity of fluorescence for Example 2 by flow cytometry.
Fig. 5

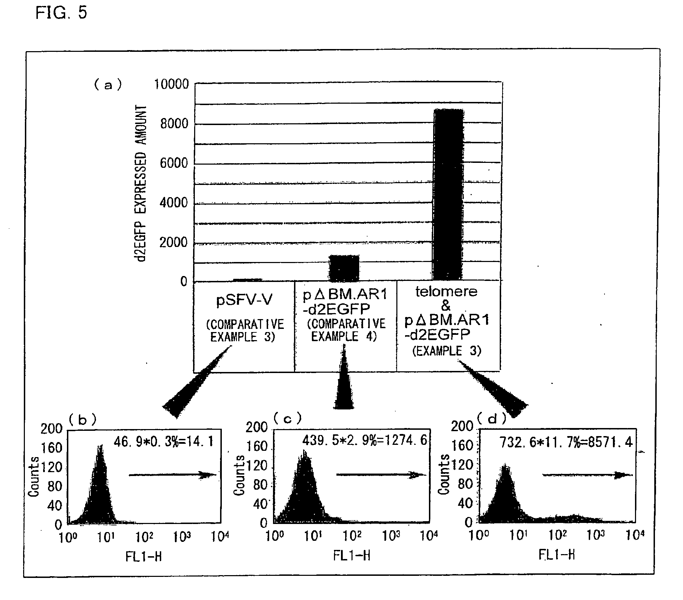

   Fig. 5 is a view illustrating a result of detecting expression of d2EGFP for polyclonal populations obtained in Comparative Example 3, Comparative Example 4, and Example 3, by flow cytometry; (a) thereof shows a result of measuring an expressed amount of d2EGFP for Comparative Example 3, Comparative Example 4, and Example 3, (b) thereof shows a result of measuring an intensity of fluorescence of d2EGFP for Comparative Example 3 by flow cytometry, (c) shows a result of measuring a fluorescence intensity of d2EGFP for Comparative Example 4 by flow cytometry, and (d) thereof shows a result of measuring an intensity of fluorescence of d2EGFP for Example 3 by flow cytometry.
Fig. 6

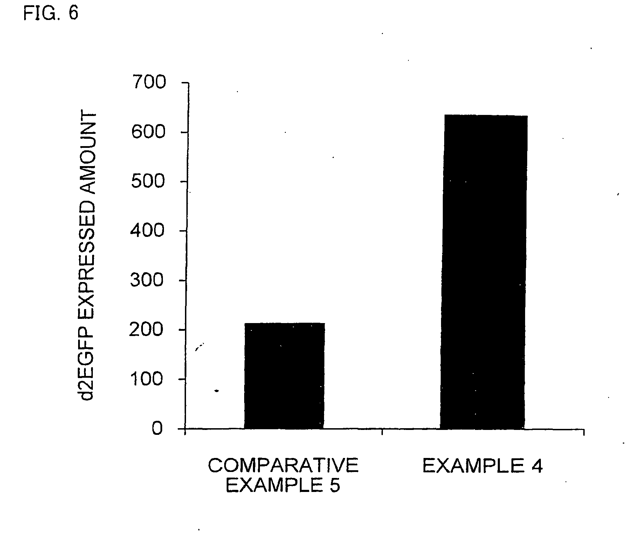

   Fig. 6 is a view showing a result of detecting an expression of d2EGFP for polyclonal populations obtained in Comparative Example 5 and Example 4 by flow cytometry.

### Description of Embodiments

Details of the present invention are described as follows. However, the present invention is not limited to the description as set forth below and may be altered within the main point of the present invention.

### < 1. Gene amplification method and target gene expression method of the present invention>

The present invention relates to a method of amplifying a target gene outside a chromosome of a mammalian cell. This method hereinafter is called "gene amplification method of the present invention". In the embodiment, "amplifying a target gene outside a chromosome of a mammalian cell" means that, by performing the gene amplification method of the present invention, a target gene is amplified on a double minute chromosome (hereinafter referred to as "DM" as appropriate) which is a chromosome outside the chromosome of a mammalian cell (i.e. host cell), and/or is amplified as the DM form, that is, outside the chromosome. How to detect whether or not the target gene is amplified on the DM outside the chromosome of the mammalian cell (i.e. host cell) and/or in the form of the DM is not particularly limited. For instance, a gene transferred to the mammalian cell can be detected by performing a publicly known FISH (fluorescence in situ hybridization) on the chromosome in a mitotic phase. This can be easily performed by a person skilled in the art. The FISH can be performed in any way and may be carried out in a publicly known manner.

The gene amplification method of the present invention includes the step of transferring, concurrently to a mammalian cell, (i) a vector (i.e. "IR/MAR vector") including (a) a mammalian replication initiation region functioning in a mammalian cell and (b) a nuclear matrix attachment region functioning in a mammalian cell, (ii) a target gene, and (iii) a polynucleotide including a telomere repetitive sequence.

In addition to the gene transferring step described above, the gene amplification method of the present invention may include the step of selecting the mammalian cell to which the target gene, the IR/MAR vector, and the telomere repetitive sequence are transferred (hereinafter called "selecting step"). The selecting step is carried out after the gene transferring step.

Moreover, by combining a step of culturing the mammalian cell selected in the selecting step (i.e. transformed cell) (hereinafter called "culturing step") with the gene amplification method of the present invention, it is possible to express a large amount of the target gene. Namely, it can be said that the present invention includes a method of expressing a target gene, including the culturing step (hereinafter referred to as "target gene expression method of the present invention").

The target gene expression method of the present invention may further include a step of purifying a target protein produced in the culturing step (hereinafter called "purifying step"). By including the purifying step in the gene expression method of the present invention, it is possible to obtain a highly pure target protein.

Each step in the gene amplification method of the present invention and the gene expression method of the present invention is described below. In the following description, a mode including the "selecting step" is described for the gene amplification method of the present invention and a mode including the "purifying step" is described for the target gene expression method of the present invention. However, the present invention is not limited to these modes, and the present invention does not necessarily need to include the "selecting step" or "purifying step".

### 1-1. Gene transferring step

The step of transferring a gene in the gene amplification method of the present invention is a step which concurrently transfers, to a mammalian cell, (i) a vector (i.e. "IR/MAR vector") including (a) a mammalian replication initiation region which functions in a mammalian cell and (b) a nuclear matrix attachment region which functions in a mammalian cell, (ii) a target gene, and (iii) a polynucleotide including a telomere repetitive sequence.

The mammalian replication initiation region and mammalian nuclear matrix attachment region, which are contained in the IR/MAR vector, are not particularly limited as long as they are a replication initiation region and a nuclear matrix attachment region which function within a mammalian cell. Examples of the mammalian replication initiation region include mammalian replication initiation regions derived from a c-myc locus, a dihydrofolate reductase (DHFR) locus, or a β-globin locus. For details of the replication initiation region derived from the c-myc locus, see reference literature "McWhinney, C. et al., Nucleic Acids Res. vol. 18, p1233-1242 (1990)". For details of the replication initiation region of the dihydrofolate reductase locus, see reference literature "Dijkwel, P.A. et al., Mol. Cell. Biol. vol.8, p5398-5409 (1988)". For details of the replication initiation region of the β-globin locus, see reference literature "Aladjem, M. et al., Science vol. 281, p1005-1009 (1998)".

Moreover, examples of the nuclear matrix attachment region include polynucleotides derived from nuclear matrix attachment regions of an Igκ locus, an SV40 early region, and a dihydrofolate reductase locus. For details of the nuclear matrix attachment region of the Igκ locus, see reference literature "Tsutsui, K. et al., J. Biol. Chem. vol. 268, p12886-12894 (1993)". For details of the nuclear matrix attachment region of the SV40 early region, see reference literature "Pommier, Y. et al., J. Virol., vol 64, p419-423 (1990)". For details of the nuclear matrix attachment region of the dihydrofolate reductase locus, see "Shimizu N. et al., Cancer Res. vol. 61, p6987-6990". As to the IR/MAR vector, reference can be made to Patent Literature and Non Patent Literature raised in Background Art.

With the IR/MAR vector, the IR/MAR vector may include (a) a sequence required for cloning within *Escherichia coli,* (b) a selective marker gene such as a drug resistance gene (e.g. blasticidin resistant gene, neomycin resistant gene, hygromycin resistant gene) or a green fluorescence protein gene, and (c) a purifying tag gene such as a histidine tag. The selective marker functions as an indicator so as to allow selecting a mammalian cell to which the IR/MAR vector was transferred. That is to say, by including the selective marker gene in the IR/MAR vector, it is easily possible to carry out the selecting step described later. Moreover, in a case where the IR/MAR vector includes the target gene and the vector is designed so that the target protein is expressed together with a purifying tag, it is easily possible to carry out the purifying step described later.

Moreover, the polynucleotide including the telomere repetitive sequence (also simply called "telomere sequence") may be a polynucleotide made just of the telomere repetitive sequence, or may be a polynucleotide in which the telomere repetitive sequence is ligated with any other nucleotide chain. The telomere repetitive sequence is known as a minisatellite present in a telomere at an end of the chromosome, and its sequence is said to be similar beyond its species. It is known with humans that several thousand base pairs of a repetitive sequence whose repeating unit is TTAGGG is present on a chromosome end. The telomere repetitive sequence in the present invention is meant to include all base sequences known as a telomere repetitive sequence, and its base sequence is not particularly limited. The sequence of the telomere slightly differs depending on the species. For example, in a case of vertebrates such as humans, mice, rats and the like, the repeating unit thereof is the aforementioned TTAGGG. Meanwhile, nematodes (C. elegans) have a repeating unit of TTAGGC, insect silkworms have a repeating unit of TTAGG, the plant Arabidopsis thaliana has a repeating unit of TTTAGGG, and budding yeast has a sequence in which TG, TGG, and TGGG are randomly repeated. The telomere repetitive sequence that is employed in the present invention may be any telomere repetitive sequence as long as a base sequence of the aforementioned repeating unit is repeated several times. The telomere repetitive sequence employed in the present invention may be a sequence in which one base is substituted, lacked or added in the base sequence of the repeating unit. Furthermore, this repeated number is not particularly limited as long as the effect of the present invention (see description under Effect of the Invention) is brought about. The number of repeated times in the telomere repetitive sequence that is employable in the present invention can be determined by preparing various telomere repetitive sequences and confirming whether or not the effect of the present invention is brought about with use of the prepared telomere repetitive sequences.

The polynucleotide including the telomere repetitive sequence employed in the present invention is not particularly limited in its size, however it is preferable that the size thereof is within a range of not less than 500 bp to not more than 10,000 bp (500 bp to 10,000 bp). By having the size of the polynucleotide including the telomere repetitive sequence be within the preferable range, it is possible to achieve a high transfer efficiency to the host cell of the polynucleotide including the telomere repetitive sequence, the target gene, and the IR/MAR vector, and further makes it easier to bring about the effect of the present invention (effect of amplifying the target gene outside the chromosome of the mammalian cell) with high efficiency. The telomere repetitive sequence is obtainable by PCR or by chemical synthesis, since its base sequence is already known. In the Example later described, the polynucleotide including the telomere sequence is obtained by PCR.

The term "target gene" means a polynucleotide that encodes a protein to be expressed. The target gene may be DNA or RNA. The target gene is not particularly limited, and may be selected as needed from any polynucleotide that encodes a desired protein. The polynucleotide, which is the target gene, may be obtained by a publicly known technique such as PCR, based on information of the base sequence of the target gene.

It is preferable that the target gene be controllably ligated to a promoter. The promoter is not particularly limited, and may be selected from those that function in the mammalian cell, to which the promoter is to be transferred. The promoter may be a promoter in which a transcription activity thereof can be activated or inactivated with a prescribed operation by a transcription factor etc. (hereinafter, referred to as "transcription activity-adjusting promoter"), or may be a homeostatic promoter in which the transcription activity is homeostatically activated. The "transcription activity-adjusting promoter" is not particularly limited and may be selected from those having the above characteristics. For example, commercially available products such as TRE promoter (manufactured by Clontech Laboratories Inc.) and T-REX promoter (manufactured by Invitrogen Corporation) are applicable in the method according to the present invention. Examples of the applicable homeostatic promoter are a CMV promoter, a SV40 promoter, an SRalpha promoter, an LTR promoter, an MMTV promoter, and the like.

In the gene transferring step of the gene amplification method according to the present invention, the IR/MAR vector, the target gene, and the polynucleotide including the telomere repetitive sequence are concurrently transferred to the mammalian cell (also called "mammal-derived cell"). It is well known that the mammalian cell is optimum for producing useful protein for drugs and the like, and the present invention is also preferably usable as means for producing the useful protein for drugs and the like.

The mammalian cell is not particularly limited, and may be a tumor cell such as human colon COLO 320DM tumor cell (available from ATCC CCL-220 etc.) or the like, or various cells such as a CHO-K1 cell (available from ATCC CCL-61, RIKEN RCB0285, RIKEN RCB0403 etc.). However, there are cases where a tumor cell having infinite proliferation ability is preferable as the mammalian cell. Examples of the tumor cell are a HeLa cell (available from ATCC CCL-2, ATCC CCL-2.2, RIKEN RCB0007, RIKEN RCB0191 etc.), human colon COLO 320HSR tumor cell (available from ATCC CCL-220.1), and NS0 cell (available from RIKEN RCB0213). For details of the human colon COLO 320DM tumor cell, see "Shimizu, N.et al. Nat. Genet., 12: 65-71, 1996.".

In transferring the IR/MAR vector, the target gene, and the polynucleotide including the telomere repetitive sequence to the mammalian cell, the transferring is not particularly limited as long as all of the IR/MAR vector, the target gene, and the polynucleotide are concurrently transferred to the mammalian cell. For instance, the IR/MAR vector, the target gene, and the polynucleotide including the telomere repetitive sequence may either be connected with each other so as to be transferred to the mammalian cell as a same gene construct, or the IR/MAR vector, the target gene, and the polynucleotide including the telomere repetitive sequence may be transferred respectively as different gene constructs. Moreover, any selected two of the IR/MAR vector, the target gene, and the polynucleotide including the telomere repetitive sequence may be connected with each other so as to be transferred as a same gene construct, and the remaining one be transferred as a different gene construct. Particularly, it is known in a relationship between the IR/MAR vector and the target gene that the target gene is highly amplifiable in either case of (i) the IR/MAR vector and the target gene that are connected to each other being transferred to the host cell as a same gene construct or (ii) the IR/MAR vector and the target gene being transferred to the host cell as different gene constructs. Accordingly, any of the aforementioned modes can be employed. In the embodiment, the transferring to the host cell of the IR/MAR vector and the target gene that are connected to each other as a same gene construct is referred to as "arranging the IR/MAR vector and the target gene in cis", whereas the transferring to the host cell of the IR/MAR vector and the target gene as separate gene constructs is referred to as "arranging the IR/MAR vector and the target gene in trans". The case of "arranging the IR/MAR vector and the target gene in cis" has such an advantage that operation is easy because the number of gene constructs is reduced in number. On the other hand, the case of "arranging the IR/MAR vector and the target gene in trans" allows for reducing each size of the gene construct. This is advantageous in that a high gene transfer efficiency is achievable. In the case of transferring to the mammalian cell the IR/MAR vector, the target gene, and the polynucleotide including the telomere repetitive sequence as separate gene constructs, and in the case where any two selected from the group consisting of the IR/MAR vector, the target gene, and the polynucleotide including the telomere repetitive sequence are connected to form a same gene construct and the remaining one is transferred as a separate gene construct, a mixture ratio of each of the gene constructs is not particularly limited as long as it is within a range in which the effect of the present invention can be brought about, and is a matter employable upon consideration of a preferable condition by a person skilled in the art as appropriate.

Structure of the gene construct may either be a plasmid or a cosmid. Further, a method for transferring the IR/MAR vector, the target gene, and the telomere repetitive sequence to the mammalian cell is not particularly limited, and may be selected from publicly known methods such as a lipofection, electroporation method, and particle gun method, as needed.

In the case where the IR/MAR vector and the target gene are transferred to the mammalian cell by arranging the IR/MAR vector and the target gene in trans, it is preferable that each of gene constructs include a selective marker gene. This allows for easily selecting the transformed cell to which the gene constructs are respectively transferred. At this time, it is preferable that the selective marker genes included in the gene constructs be different from each other.

### 1-2. Selecting step

The "selecting step" of the gene amplification method of the present invention is a step of selecting the mammalian cell to which the IR/MAR vector, the target gene, and the polynucleotide including the telomere repetitive sequence are transferred. More specifically, the present step is a step of selecting the mammalian cells to which the IR/MAR vector, the target gene, and the polynucleotide including the telomere repetitive sequence are transferred, from a polyclonal population including mammalian cells to which one or more of the IR/MAR vector, target gene, and polynucleotide including the telomere repetitive sequence is not transferred.

The selecting step can be easily performed by utilizing a selective marker gene such as a drug resistance gene. For instance, if the gene construct used in transferring the IR/MAR vector, the target gene, and the polynucleotide including the telomere repetitive sequence to the mammalian cell includes a drug resistance gene, the drug resistance may be used for selecting the mammalian cells, to which the IR/MAR vector, the target gene, and the polynucleotide including the telomere repetitive sequence are transferred. In the case where the selecting step is carried out by utilizing the drug resistance as an indicator, a step of culturing the mammalian cell in a culture may be included. This step cultures a mixture of a mammalian cell not including one or more of the IR/MAR vector, the target gene, and the polynucleotide including the telomere repetitive sequence and a mammalian cell to which all of the IR/MAR vector, the target gene, and the polynucleotide including the telomere repetitive sequence. In contrast, the culturing step (later described) cultures a cell already selected as a mammalian cell to which all of the IR/MAR vector, the target gene, and the polynucleotide including the telomere repetitive sequence are cultured. Consequently, the two steps are clearly distinguished from each other.

Further, the selecting step in the gene amplification method of the present invention can be carried out also by detecting, through PCR or Southern blotting, the IR/MAR vector, the target gene, and the nucleotide chain of the polynucleotide including the telomere repetitive sequence, which are included in the mammalian cell. The method of utilizing the drug resistance as an indicator, the method of utilizing PCR, and the method of utilizing the Southern blotting are not particularly limited in more specific details, and may be adopted in a publicly known manner as needed.

### 1-3. Culturing step

The "culturing step" in the target gene expression method of the present invention is a step of culturing the mammalian cells which have already been selected in the selecting step (i.e., "transformed cell"). The culturing step makes it possible to proliferate transformed cells in which a target gene is amplified outside the chromosome of the mammalian cell. Further, by expressing a bulk amount of target genes by a prescribed operation (e.g., transcription-inducing operation), it is possible to produce a bulk amount of target proteins. The present invention relates to a method for amplifying a target gene outside the chromosome of the mammalian cell.

Specific details of the culturing step is not particularly limited, and may be carried out in any way in consideration of an optimum condition for the mammalian cells to be cultured.

Particularly, the inventor of the present invention already has found that an expressed amount of the target gene can be further improved by including, in a culture medium used in the culturing step, at least one of a histone deacetylase inhibitor and a DNA methylation inhibitor (see Patent Literature 9). Hence, in order to further achieve high-level expression of the target gene by culturing the transformed cell, it can be said as preferable to include, in the culture medium, one or more of the histone deacetylase inhibitor and the DNA methylation inhibitor. The target gene can be caused to carry out high-level expression by releasing the epigenetic expression inhibition on the target gene, which releasing is achieved by the histone deacetylase inhibitor raising the acetylated level of histone and by the DNA methylation inhibitor reducing the DNA methylation level.

The histone deacetylase inhibitor and the DNA methylation inhibitor are not particularly limited. Examples of the histone deacetylase inhibitor are butyrate, Trichostatin A (TSA), MS-275, Oxamflatin, DMSO, and the like, and 5-aza-2'-deoxycytidine, 5-aza-2'-cytidine, and the like are examples of the DNA methylation inhibitor.

The amount added of the inhibitors to the culture medium can be employed within a range which does not effect the proliferation of the cultured transformed cells, upon consideration of an added amount which improves the expressed amount of the target gene.

### 1-4. Purifying step

The "purifying step" in the present invention is a step of purifying the target protein, which target protein is produced in the culturing step.

More specifically, the protein purification in the purifying step includes, for example, suspending the mammalian cells in a buffer solution such as PBS (Phosphate Buffered Saline); breaking the cells with use of a homogenizer, ultrasonic wave, or the like; centrifugalizing the solution; and collecting a supernatant liquid. To the buffer solution, the followings may also be added as needed: a surfactant for promoting solubilization of the protein; a reducing agent for stabilizing a conformation of the protein; and a protease inhibitor for inhibiting degradation of the protein. Examples of the surfactant are CHAPS (3-[(3-cholamidopropyl)-dimethylammonio-1-propanesulfonate]), Triton X-100, Nikkol, and n-octyl glucoside. Examples of the reducing agent are DTT (dithiothreitol) and DET (dithioerythritol). Examples of the protease inhibitor are aprotinin and leupeptine.

A target protein can be purified from the supernatant liquid, with use of a column chromatography such as an affinity chromatography, ion exchanging chromatography, and filtration chromatography. It is also possible to remove unwanted salt by dialyzing the purified protein solution against an adequate buffer solution. The step for purifying the protein is carried out preferably at a low temperature in order to inhibit degradation of the protein. More preferably, the step is carried out preferably at a temperature of 4 °C. Note that the specific method of the purifying step is not subject to this limitation, and publicly known methods can be used as appropriate.

### <2. Kit of the present invention>

The present invention further encompasses a kit for performing the above-mentioned gene amplification method of the present invention (hereinafter called "kit of the present invention"). The kit of the present invention includes: a vector including (a) a mammalian replication initiation region functioning in a mammalian cell and (b) a nuclear matrix attachment region functioning in a mammalian cell (IR/MAR vector); and a polynucleotide including a telomere repetitive sequence.

The description in <1. Gene amplification method of the present invention> can be incorporated as the description regarding the configuration of a kit of the present invention. The kit according to the present invention is not particularly limited to the above configuration as long as the kit can perform the gene amplification method according to the present invention, and may include other configurations. For example, the kit of the present invention may include a buffer solution required for transformation, a restriction enzyme for inserting the target gene into the vector, a vial, tubes, and the like. Note that by including at least one or more of the histone deacetylase inhibitor and DNA methylation inhibitor in the kit of the present invention, it is possible to configure a target gene expression kit that allows further high-level expression of the expressed amount of genes.

Described below are Examples further describing the embodiment of the present invention in detail. Of course, the present invention is not limited by the following Examples, and it is needless to say that various modes are applicable for detailed parts thereof.

### Examples

### <Experiment 1>

### Method

A double stranded DNA consisting of a telomere repetitive sequence was prepared by the method schematically described in Fig. 1. Namely, a single stranded DNA of 24 mer in which 5'-TTAGGG-3' is repeated four times and a single stranded DNA of 24 mer in which 5'-CCCTAA-3' is repeated four times were chemically synthesized and were mixed. PCR reaction was carried out by having the two types of single stranded DNA serve respectively as a primer and a template, to prepare a double stranded DNA (double stranded DNA consisting of a telomere repetitive sequence) having a chain length distribution of about 500 bp to about 10,000 bp and having an average chain length of about 1200 bp. The repeating unit TTAGGG is a repeating unit in a telomere repetitive sequence derived from vertebrates such as humans, mice, rats and the like.

Meanwhile, Fig. 2 illustrates a structure of pΔBM.AR1-d2EGFP, which is an IR/MAR vector. This plasmid was constructed by the following method. First, pΔB.AR1 was cleaved at Bam HI/Mlu I and a Hyg gene region was removed. To this cleavage site, a MCS (multiple cloning site) having Asc I and Asis I cleavage sites was inserted, by which a plasmid (pΔBM.AR1-MCS) was constructed. Next, the pΔBM.AR1-MCS was cleaved at Asc I and Asis I, and a d2EGFP gene expression cassette was inserted to the cleavage site to construct pΔBM.AR1-d2EGFP. As a comparative plasmid, a plasmid (pSFV-V) not having IR and MAR was used. The pΔB.AR1 is disclosed in Patent Literature 5 (Publication No. 2006-320332 A) and the pSFV-V plasmid is disclosed in reference literature: N. Shimizu et al., Cancer Research (2003).

A mixture of DNA consisting of telomere repetitive sequence having an average of 1200 bp and ΔBM.AR1-d2EGFP plasmid DNA was prepared with a different mixture ratio, and was transferred to human colon COLO 320DM tumor cell or a Chinese hamster CHO-K1 cell, by use of the lipofection method. Utilizing that the plasmid holds a Blasticidin resistance gene, a polyclonal population with a stable transformant was obtained by culturing for approximately 1 month in the presence of 10 µg/ml of Blasticidin. The culturing of the human colon COLO 320DM tumor cell used RPMI 1640 culture medium (manufactured by Nissui Pharmaceutical Co., Ltd.), and the culturing of the Chinese hamster CHO-K1 cell used Ham F12 culture medium (manufactured by Nissui Pharmaceutical Co., Ltd.) (hereinafter same).

From the polyclonal population obtained from the COLO 320DM cell, a metaphase chromosome sample was prepared. FISH (fluorescence in situ hybridization) was performed with use of a probe prepared from the transferred plasmid DNA, to detect a base sequence of the transferred plasmid.

### Result

The following describes results of comparing frequencies of various gene amplification forms (HSR, ND) included in various polyclonal populations as a result of subjecting the polyclonal populations to the FISH, which polyclonal populations were prepared by transferring a mixture of DNA consisting of telomere repetitive sequence (for convenience, called "telomere DNA") and pΔBM.AR1-d2EGFP plasmid DNA, with different mixture ratios, to human colon COLO 320DM tumor cells.

In Fig. 3, "none" represents a proportion of clones in which the transferred DNA was not amplified, "HSR" represents a proportion of clones in which the transferred gene was amplified in the form of the HSR, and "DM" represents a proportion of clones in which the transferred gene was amplified in the form of the DM. Moreover, in Fig. 3, "pΔBM.AR1-d2EGFP" shows a result of a polyclonal population to which just pΔBM.AR1-d2EGFP was transferred to the human colon COLO 320DM tumor cell (Comparative Example 1), "pΔBM.AR1-d2EGFP & telomere 0.3 µg" shows a result of a polyclonal population to which a mixture in which 0.3 µg of telomere DNA was mixed to 1.0 µg of pΔBM.AR1-d2EGFP was transferred to the human colon COLO 320DM tumor cell (Example 1), "pΔBM.AR1-d2EGFP & telomere 1.0 µg" shows a result of a polyclonal population to which a mixture in which 1.0 µg of telomere DNA was mixed to 1.0 µg of pΔBM.AR1-d2EGFP was transferred to the human colon COLO 320DM tumor cell (Example 2), and "pSFV-V & telomere 1.0 pg" shows a result of a polyclonal population to which a mixture in which 1.0 µg of telomere DNA was mixed to 1.0 µg of pSFV-V (a plasmid including no IR or MAR) was transferred to the human colon COLO 320DM tumor cell (Comparative Example 2).

According to Fig. 3, it was observed that in a case where the pΔBM.AR1-d2EGFP plasmid DNA was solely transferred to the human colon COLO 320DM tumor cell, the base sequence of the plasmid was efficiently amplified in the form of the HSR or DM. On the other hand, in a case where the telomere DNA was transferred concurrently with the pΔBM.AR1-d2EGFP plasmid, the formation of the HSR was remarkably inhibited, and instead cells amplified in the form of the DM increased (Examples 1 and 2). Note that in the case where the pSFV-V, which is not an IR/MAR vector, was solely transferred to the human colon COLO 320DM tumor cell, hardly any gene amplification occurred (Comparative Example 2).

### <Experiment 2>

### Method

Expression of d2EGFP was studied by flow cytometry, for the polyclonal populations obtained in Comparative Example 1, and Examples 1 and 2. The d2EGFP is a decay 2 enhanced green fluorescence protein, and encodes a green fluorescent protein which has an extremely short intracellular half-life period. This gene is included in pΔBM.AR1-d2EGFP plasmid, and so by measuring the green fluorescence of the cell, it is possible to measure the amount of protein produced per cell at that point.

### Result

A result thereof is shown in Fig. 4. In Fig. 4, (a) shows a result of measuring an expressed amount of d2EGFP for Comparative Example 1, and Examples 1 and 2, (b) shows a result of measuring an intensity of fluorescence of d2EGFP by flow cytometry for Comparative Example 1, (c) shows a result of measuring an intensity of fluorescence of d2EGFP by flow cytometry for Example 1, and (d) shows a result of measuring an intensity of fluorescence of d2EGFP by flow cytometry, for Example 2.

As illustrated in Fig. 4, the expressed amount of d2EGFP largely increased by concurrently transferring the telomere DNA and the pΔBM.AR1-d2EGFP plasmid (Example 1 obtained a result 13.4 times more of that of Comparative Example 1. Example 2 obtained a result 4.7 times more of that of Comparative Example 1).

### <Experiment 3>

### Method

To 1.0 µg of pΔBM.AR1-d2EGFP, 1.0 µg of the telomere DNA prepared in Experiment 1 was mixed, and this mixture was transferred to a Chinese hamster CHO-K1 cell by use of the lipofection method. Utilizing that the plasmid holds a Blasticidin resistance gene, a polyclonal population of a stable transformant was obtained by culturing for approximately 1 month in the presence of 10 µg/ml Blasticidin (Example 3). For comparison, a polyclonal population was obtained by transferring pSFV-V, which is a plasmid including no IR or MAR, to a Chinese hamster CHO-K1 cell, as similar to the above (Comparative Example 3). Moreover, a polyclonal population was obtained by transferring just the pΔBM.AR1-d2EGFP to the Chinese hamster CHO-K1 cell, as similarly to the above (Comparative Example 4).

Expression of d2EGFP was studied by flow cytometry as with Experiment 2, for each of the polyclonal populations obtained in Comparative Example 3, Comparative Example 4, and Example 3.

### Result

A result thereof is shown in Fig. 5. In Fig. 5, (a) shows a result of measuring an expressed amount of d2EGFP for Comparative Example 3, Comparative Example 4, and Example 3, (b) shows a result of measuring an intensity of fluorescence of d2EGFP by flow cytometry for Comparative Example 3, (c) shows a result of measuring an intensity of fluorescence of d2EGFP by flow cytometry for Comparative Example 4, and (d) shows a result of measuring an intensity of fluorescence of d2EGFP by flow cytometry for Example 3.

As illustrated in Fig. 5, the expressed amount of d2EGFP largely increased by concurrently transferring the telomere DNA and the pΔBM.AR1-d2EGFP plasmid, even in a case where the CHO-K1 cell is used as the host cell (Example 3 obtained a result 608.0 times more of that of Comparative Example 3, and obtained a result 6.7 times more of that of Comparative Example 4).

### <Experiment 4>

### Method

A telomere DNA holding a repeating unit TTAGGC of a telomere repetitive sequence derived from a nematode (C. elegans) was prepared in accordance with the method schematically described in Fig. 1. Namely, a single stranded DNA of 24 mer in which 5'-TTAGGC-3' is repeated four times and a single stranded DNA of 24 mer in which 5'-GCCTAA-3' is repeated four times were chemically synthesized and were mixed. PCR reaction was carried out by having the two types of single stranded DNA respectively serve as a primer and a template, to prepare a double stranded DNA (double stranded DNA made of telomere repetitive sequence) having a chain length distribution of about 500 bp to about 10,000 bp and having an average chain length of about 1200 bp. The repeating unit of the telomere repetitive sequence derived from the nematode (C. elegans) and the repeating sequence of the telomere repetitive sequence prepared in Experiment 1 which were derived from vertebrates such as humans, mice, rats and the like, are in relationship of a single base substitution.

To 2.0 µg of pΔBM.AR1-d2EGFP, 1.0 pg of telomere DNA derived from the nematode prepared as described above was mixed, and this mixture was transferred to the human colon COLO 320DM tumor cell by use of the lipofection method. Utilizing that the plasmid holds a Blasticidin resistance gene, a polyclonal population of a stable transformant was obtained by culturing for approximately 1 month in the presence of 10 µg/ml Blasticidin (Example 4).

Moreover, a polyclonal population was obtained by transferring just 2.0 µg of pΔBM.AR1-d2EGFP to the human colon COLO 320DM cell, as described above (Comparative Example 5).

Expression of d2EGFP was studied by flow cytometry as with Experiment 2, for each of various polyclonal populations (cells of logarithmic breeding period) obtained in Example 4 and Comparative Example 5.

### Result

Fig. 6 illustrates a result of measuring a d2EGFP expressed amount for Comparative Example 5 and Example 4.

As illustrated in Fig. 6, it was confirmed that the expressed amount of d2EGFP largely increased even by concurrently transferring the telomere DNA derived from a nematode and pΔBM.AR1-d2EGFP which is a IR/MAR vector (Example 4 yielded a result approximately three times more of that of Comparative Example 5).

This result demonstrates that even if the telomere sequence were a telomere sequence other than the telomere sequence derived from the vertebrate as was used in Experiments 1 to 3, such a telomere sequence can be used in the present invention.

### Industrial Applicability

As described above, according to the present invention, it is possible to amplify a target gene in a mammalian cell in the form of a DM, with high efficiency. Hence, together with the amplification of the target gene, it is possible to achieve a merit that the amount of target protein expressed from the target gene also increases.

This as a result allows for establishing a bulk production system of target protein, which further improves the conventional high gene amplification system. Therefore, the present invention provides such an advantage that a bulk amount of target proteins (e.g., protein useful as drugs) can be produced as compared to the conventional technique.

Accordingly, the present invention is applicable in a wide range of industries which involve protein production, such as pharmaceutical, chemical, food, cosmetic, and textile industries.

## Claims

1. A method of amplifying a target gene outside a chromosome of a mammalian cell, the method comprising the step of:
transferring, concurrently to a mammalian cell, (i) a vector including (a) a mammalian replication initiation region functioning in a mammalian cell and (b) a nuclear matrix attachment region functioning in a mammalian cell, (ii) a target gene, and (iii) a polynucleotide including a telomere repetitive sequence, wherein the polynucleotide including a telomere repetitive sequence has a size within a range of not less than 500 bp to not more than 10,000 bp.

2. The method according to claim 1, wherein the telomere repetitive sequence is a base sequence consisting of a base sequence of TTAGGG or TTAGGC being repeated several times.

3. The method according to claim 1 or claim 2, wherein the mammalian replication initiation region derives from a replication initiation region of a locus selected from the group consisting of a c-myc locus, a dihydrofolate reductase locus, and a β-globin locus.

4. The method according to any one of claims 1 to 3, wherein the nuclear matrix attachment region derives from a nuclear matrix attachment region of a region selected from the group consisting of an Igκ locus, an SV40 early region, and a dihydrofolate reductase locus.

5. The method according to any one of claims 1 to 4, wherein the target gene and the vector are transferred to the mammalian cell in such a manner that the target gene and the vector are arranged in cis.

6. The method according to any one of claims 1 to 4, wherein the target gene and the vector are transferred to the mammalian cell in such a manner that the target gene and the vector are arranged in trans.

7. The method according to any one of claims 1 to 6, further comprising the step of:
selecting a transformed cell after the step of transferring.

8. A transformed cell being a mammalian cell to which (i) a vector including (a) a mammalian replication initiation region functioning in a mammalian cell and (b) a nuclear matrix attachment region functioning in a mammalian cell, (ii) a target gene, and (iii) a polynucleotide including a telomere repetitive sequence are concurrently transferred, the target gene being amplified outside a chromosome of the mammalian cell, wherein the polynucleotide including a telomere repetitive has a size within a range of not less than 500 bp to not more than 10,000 bp, and wherein the transformed cell comprises the vector (i), the target gene (ii), and the polynucleotide (iii).

9. The transformed cell according to claim 8, wherein the telomere repetitive sequence is a base sequence consisting of a base sequence of TTAGGG or TTAGGC being repeated several times.

10. The transformed cell according to claim 8 or 9, wherein the mammalian replication initiation region derives from a replication initiation region of a locus selected from the group consisting of a c-myc locus, a dehydrofolate reductase locus, and a β-globin locus.

11. The transformed cell according to any one of claims 8 to 10, wherein the nuclear matrix attachment region derives from a nuclear matrix attachment region of a region selected from the group consisting of an Igκ locus, an SV40 early region, and a dihydrofolate reductase locus.

12. A method of expressing a target gene, comprising the step of:
culturing a transformed cell as set forth in any one of claims 8 to 11.

13. A kit for amplifying a target gene outside a chromosome of a mammalian cell, the kit comprising:
a vector including (a) a mammalian replication initiation region functioning in a mammalian cell and (b) a nuclear matrix attachment region functioning in a mammalian cell; and
a polynucleotide including a telomere repetitive sequence, wherein the polynucleotide including a telomere repetitive sequence has a size within a range of not less than 500 bp to not more than 10,000 bp.

14. The kit according to claim 13, wherein the telomere repetitive sequence is a base sequence consisting of a base sequence of TTAGGG or TTAGGC being repeated several times.

15. The kit according to claim 13 or claim 14, wherein the mammalian replication initiation region derives from a replication initiation region of a locus selected from the group consisting of a c-myc locus, a dihydrofolate reductase locus, and a β-globin locus.

16. The kit according to any one of claims 13 to 15, wherein the nuclear matrix attachment region derives from a nuclear matrix attachment region of a region selected from the group consisting of an Igκ locus, an SV 40 early region, and a dihydrofolate reductase locus.

## Patentansprüche

1. Verfahren zum Amplifizieren eines Zielgens außerhalb eines Chromosoms einer Säugerzelle, das Verfahren umfassend den Schrift:
Gleichzeitiges Übertragen an eine Säugerzelle von (i) einem Vektor enthaltend (a) eine in einer Säugerzelle funktionierende Säugerreplikationsinitiationsregion und (b) eine in einer Säugerzelle funktionierende Kernmatrixanheftungsregion, (ii) einem Zieigen, und (iii) einem eine repetitive Telomersequenz enthaltenden Polynukleotid, wobei das eine repetitive Telomersequenz enthaltende Polynukleotid eine Größe in einem Bereich von nicht weniger als 500 bp bis nicht mehr als 10000 bp aufweist.

2. Verfahren nach Anspruch 1, wobei die repetitive Telomersequenz eine Basensequenz ist, die aus einer Basensequenz TTAGGC oder TTAGGC, die mehrfach wiederholt ist, besteht.

3. Verfahren nach Anspruch oder 2. wobei die Säugerreptikationsinitiationsregion von einer Replikationsinitiationsregion eines Locus, der ausgewählt ist aus der Gruppe bestehend aus einem c-myc-Locus, einem Dihydrofolatreduktase-Locus, und einem β-Globin-Locus, stammt.

4. Verfahren nach einem der Ansprüche 1 bis 3, wobei die Kemmatrixanheftungsregion von einer Kernmatrixanheftungsregion einer Region, die ausgewählt ist aus der Gruppe bestehend aus einem Igκ-Locus, einer SV40-frühen Region, und einem Dihydrofolatreduktase-Locus, stammt.

5. Verfahren nach einem der Ansprüche 1 bis 4, wobei das Zielgen und der Vektor auf eine solche Weise an die Säugerzelle übertragen sind, dass das Zielgen und der Vektor in cis angeordnet sind.

6. Verfahren nach einem der Ansprüche 1 bis 4, wobei das Zielgen und der Vektor auf eine solche Weise an die Säugerzelle übertragen sind, dass das Zielgen und der Vektor in trans angeordnet sind.

7. Verfahren nach einem der Ansprüche 1 bis 6, weiterhin umfassend den Schritt:
Auswählen einer transformierten Zelle nach dem Schritt des Übertragens.

8. Transformierte Zelle, die eine Säugerzelle ist, umfassend
(i) einen Vektor enthaltend (a) eine in einer Säugerzelle funktionierende Säugerreplikationsinitiationsregion und (b) eine in einer Säugerzelle funktionierende Kernmatrixanheftungsregion,
(ii) ein Zielgen, und
(iii) ein eine repetitive Telomersequenz enthaltendes Polynukleotid, wobei dieses eine repetitive Tetomerscquenz enthaltende Polynukleotid eine Größe in einem Bereich von nicht weniger als 500 bp bis nicht mehr als 10000 bp aufweist.

9. Transformierte Zelle nach Anspruch 8. wobei die repetitive Telomersequenz eine Basensequenz ist. die aus einer Basensequenz TTAGGG oder TTAGGG, die mehrfach wiederholt ist, besteht.

10. Transformierte Zelle nach Anspruch 8 oder 9, wobei die Säugerreplikationsinitiationsregion von einer Replikationsinitiationsregion eines Locus, der ausgewählt ist aus der Gruppe bestehend aus einem c-myc-Locus. einem Dihydrofolatreduktase-Locus, und einem β-Globin-Locus, stammt.

11. Transformierte Zelle nach einem der Ansprüche 8 bis 10, wobei die Kernmatrixanheftungsregion von einer Kernmatrixanheftungsregion einer Region, die ausgewählt ist aus der Gruppe bestehend aus einem Igκ-Locus, einer SV40-frühen Region, und einem Dihydrofolatreduktase-Locus, stammt.

12. Verfahren zum Exprimieren eines Zielgens umfassend den Schritt:
Kultivierten einer transfortnierten Zelle nach einem der Ansprüche 8 bis 11.

13. Kit zum Amplifizieren eines Zielgens außerhalb eines Chromosoms einer Säugerzelle, das Kit umfassend:
einen Vektor enthaltend (a) eine in einer Säugerzelle funktionierende Säugerreplikationsinitiationsregion und (b) eine in einer Säugerzelle funktionierende Kernmatrixanheftungsregion; und
ein eine repetitive Telomersequenz enthaltendes Polynukleotid, wobei das eine repetitive Telomersequenz enthaltende Polynukleotid eine Größe in einem Bereich von nicht weniger als 500 bp bis nicht mehr als 10000 bp aufweist.

14. Kit nach Anspruch 13, wobei die repetitive Telomersequenz eine Basensequenz ist, die aus einer Basensequenz TTAGGG oder TTAGGC, die mehrfach wiederholt ist, besteht.

15. Kit nach Anspruch 13 oder 14, wobei die Säugerrepfikationsinitiationsregion von einer Replikationsinitiationsregion eines Locus, der ausgewählt ist aus der Gruppe bestehend aus einem c-myc-Loucus, einem Dihydrofolatreduktase-Locus, und einem β-Globin-Locus. stammt.

16. Kit nach einem der Ansprüche 13 bis 15, wobei die Kernmatrixanheftungsregion von einer Kernmatrixanheftungsregion einer Region, die ausgewählt ist aus der Gruppe bestehend aus einem Igκ-Locus, einer SV40-frühen Region, und einem Dihydrofolatreduktase-Locus, stammt.

## Revendications

1. Procédé d'amplification d'un gène cible hors d'un chromosome d'une cellule de mammifère, le procédé comprenant l'étape suivante :
le transfert, simultanément dans une cellule de mammifère, (i) d'un vecteur contenant (a) une région d'initiation de réplication de mammifère fonctionnant dans une cellule de mammifère et (b) une région d'attachement à la matrice nucléaire fonctionnant dans une cellule de mammifère, (ii) d'un gène cible, et (iii) d'un polynucléotide contenant une séquence de répétition de télomère, dans lequel le polynucléotide contenant une séquence répétition de télomère possède une taille située dans une plage allant d'au moins 500 pb à au plus 10 000 pb.

2. Procédé selon la revendication 1, dans lequel la séquence de répétition de télomère est une séquence de bases constituée d'une séquence de bases TTAGGG ou TTAGGC répétée plusieurs fois.

3. Procédé selon la revendication 1 ou la revendication 2, dans lequel la région d'initiation de réplication de mammifère provient d'une région d'initiation de réplication d'un locus choisi dans le groupe constitué d'un locus de c-myc, d'un locus de dihydrofolate réductase et d'un locus de β-globine.

4. Procédé selon l'une quelconque des revendications 1 à 3, dans lequel la région d'attachement à la matrice nucléaire provient d'une région d'attachement à la matrice nucléaire d'une région choisie dans le groupe constitué d'un locus d'Igκ, d'une région précoce de SV40 et d'un locus de dihydrofolate réductase.

5. Procédé selon l'une quelconque des revendications 1 à 4, dans lequel le gène cible et le vecteur sont transférés dans la cellule de mammifère de telle manière que le gène cible et le vecteur soient agencés en cis.

6. Procédé selon l'une quelconque des revendications 1 à 4, dans lequel le gène cible et le vecteur sont transférés dans la cellule de mammifère de telle manière que le gène cible et le vecteur soient agencés en trans.

7. Procédé selon l'une quelconque des revendications 1 à 6, comprenant en outre l'étape suivante :
la sélection d'une cellule transformée après l'étape de transfert.

8. Cellule transformée qui est une cellule de mammifère comprenant
(i) un vecteur contenant (a) une région d'initiation de réplication de mammifère fonctionnant dans une cellule de mammifère et (b) une région d'attachement à la matrice nucléaire fonctionnant dans une cellule de mammifère,
(ii) un gène cible, et
(iii) un polynucléotide contenant une séquence de répétition de télomère, dans lequel ce polynucléotide contenant une séquence de répétition de télomère possède une taille située dans une plage allant d'au moins 500 pb à au plus 10 000 pb.

9. Cellule transformée selon la revendication 8, dans laquelle la séquence de répétition de télomère est une séquence de bases constituée d'une séquence de bases TTAGGG ou TTAGGC répétée plusieurs fois.

10. Cellule transformée selon la revendication 8 ou la revendication 9, dans laquelle la région d'initiation de réplication de mammifère provient d'une région d'initiation de réplication d'un locus choisi dans le groupe constitué d'un locus de c-myc, d'un locus de déshydrofolate réductase et d'un locus de β-globine.

11. Cellule transformée selon l'une quelconque des revendications 8 à 10, dans laquelle la région d'attachement à la matrice nucléaire provient d'une région d'attachement à la matrice nucléaire d'une région choisie dans le groupe constitué d'un locus d'Igκ, d'une région précoce de SV40 et d'un locus de dihydrofolate réductase.

12. Procédé d'expression d'un gène cible, comprenant l'étape suivante :
la culture d'une cellule transformée selon l'une quelconque des revendications 8 à 11.

13. Kit permettant d'amplifier un gène cible hors d'un chromosome d'une cellule de mammifère, le kit comprenant :
un vecteur contenant (a) une région d'initiation de réplication de mammifère fonctionnant dans une cellule de mammifère et (b) une région d'attachement à la matrice nucléaire fonctionnant dans une cellule de mammifère ; et
un polynucléotide contenant une séquence de répétition de télomère, dans lequel le polynucléotide contenant une séquence répétition de télomère possède une taille située dans une plage allant d'au moins 500 pb à au plus 10 000 pb.

14. Kit selon la revendication 13, dans lequel la séquence de répétition de télomère est une séquence de bases constituée d'une séquence de bases TTAGGG ou TTAGGC répétée plusieurs fois.

15. Kit selon la revendication 13 ou la revendication 14, dans lequel la région d'initiation de réplication de mammifère provient d'une région d'initiation de réplication d'un locus choisi dans le groupe constitué d'un locus de c-myc, d'un locus de dihydrofolate réductase et d'un locus de β-globine.

16. Kit selon l'une quelconque des revendications 13 à 15, dans lequel la région d'attachement à la matrice nucléaire provient d'une région d'attachement à la matrice nucléaire d'une région choisie dans le groupe constitué d'un locus d'Igκ, d'une région précoce de SV 40 et d'un locus de dihydrofolate réductase.
